# EUROPEAN PATENT APPLICATION

(11) **EP 4 473 926 A1**
(43) Date of publication of application: **11.12.2024**
(21) Application number: 24179166.4
(22) Date of filing: 31.05.2024
(51) Int. Cl.: A61B 18/14

(54) **DOUBLE-SIDED ENCAPSULATED PLANAR CATHETER**

(30) Priority: 02.06.2023 US 202363505764 P; 28.12.2023 US 202318398738
(71) Applicant: Biosense Webster (Israel) Ltd., 2066717 Yokneam (IL)
(72) Inventor: VAN NIEKERK, Pieter Emmelius, Irvine, 92618 (US); EBRAHIMI, Babak, Irvine, 92618 (US); BASU, Shubhayu, Irvine, 92618 (US); HENRIQUEZ, Jamie, Irvine, 92618 (US)
(74) Representative: Carpmaels & Ransford LLP

(57) **Abstract**

A multilayered end effector for a mapping catheter including a first flexible circuit, a framework generally parallel to the first flexible circuit and separated therefrom by a first orthogonal gap orthogonal to the longitudinal axis, a second flexible circuit, and a location sensing coil layer having a plurality of coils suitably oriented and preferably disposed generally parallel to the framework and separated from the framework by a second orthogonal gap. The second flexible circuit can be separated from the location sensing coil layer by a third orthogonal gap. The first orthogonal gap, the second orthogonal gap, and the third orthogonal gap can be filled with a flexible non-conductive material, and the first face of the first flexible circuit and the first face of the second flexible circuit being coated with the flexible non-conductive material.

## Description

### CROSS-REFERENCE TO RELATED APPLICATIONS

This application claims priority under 35 U.S.C. § 119 to prior filed U.S. Provisional Patent Application No. 63/478,059 (Attorney Docket No. 253757.000354 BIO6801USPSP1) filed December 30, 2022, and U.S. Provisional Patent Application No. 63/505,764 (Attorney Docket No. 253757.000380 BIO6846USPSP1) filed June 02, 2023, each of which are hereby incorporated by reference as if set forth in full herein.

### FIELD

The present invention relates generally to minimally invasive medical devices, and in particular sensing catheters, and further relates to, but not exclusively, cardiac mapping catheters.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

### SUMMARY

There is provided, in accordance with an embodiment of the present invention, a multilayered end effector for a catheter. The end effector can include a first flexible circuit, a framework, a first non-conductive flexible layer, a second flexible circuit, and a second non-conductive flexible layer. The first flexible circuit can extend along a longitudinal axis from a proximal portion to a distal portion of the end effector and can include a first face and a second face. The framework can include a first side and an opposite second side and can extend along the longitudinal axis generally parallel to the first flexible circuit and with the first side spaced apart from the first flexible circuit. The first non-conductive flexible layer can at least partially encapsulate the first flexible circuit and the framework. The second flexible circuit can be disposed on the second side of the framework and spaced apart from the framework, and the second flexible circuit can have a first face and a second face. The second non-conductive flexible layer can at least partially encapsulate the second flexible circuit and the framework.

The disclosed technology includes an end effector for a mapping catheter. The end effector can include a first flexible circuit having a first face and a second face and extending along a longitudinal axis from a proximal portion to a distal portion of the end effector, a framework extending along the longitudinal axis generally parallel to the first flexible circuit and separated from the first flexible circuit by a first orthogonal gap orthogonal to the longitudinal axis, and a second flexible circuit having a first face and a second face and extending along the longitudinal axis from the proximal portion to the distal portion. The end effector can further include a location sensing coil layer having a plurality of coils suitably oriented and preferably disposed generally parallel to the framework and separated from the framework by a second orthogonal gap. The second flexible circuit can be separated from the location sensing coil layer by a third orthogonal gap.

The disclosed technology includes an end effector for a catheter. The end effector can include a flexible circuit extending generally parallel to a longitudinal axis of the end effector, a plurality of electrodes electrically connected to the flexible circuit, a framework extending along the longitudinal axis generally parallel to the flexible circuit, and a contiguous mass of flexible non-conductive material separating the flexible circuit and the framework from one another and at least partially insulating the flexible circuit and the framework. The flexible circuit can include a first flexible circuit and a second flexible circuit.

### BRIEF DESCRIPTION OF THE DRAWINGS

FIG. 1 is a schematic pictorial illustration of a medical system including a planar catheter, in accordance with an embodiment of the present invention;
FIG. 2A is an illustration of a perspective view of an end effector, in accordance with an embodiment of the present invention;
FIG. 2B is an illustration of an exploded perspective view of the end effector of FIG. 2A;
FIG. 2C is an illustration of an exploded perspective view of an end effector, in accordance with an embodiment of the present invention;
FIG. 2D1 is an illustration of an exploded perspective view of an end effector, in accordance with an embodiment of the present invention;

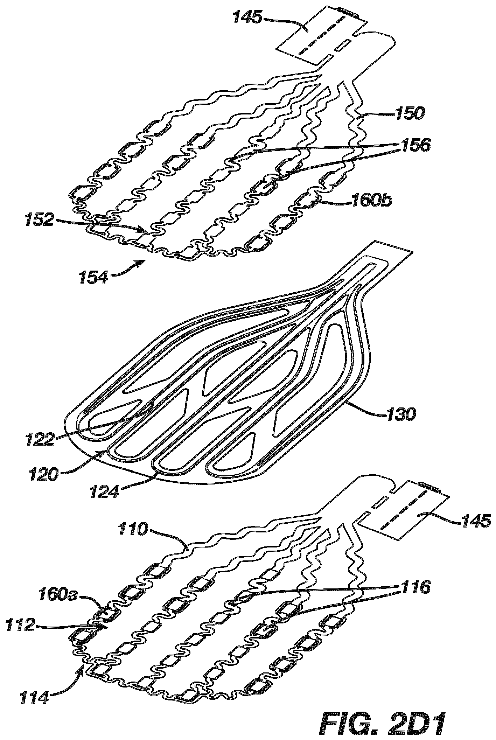

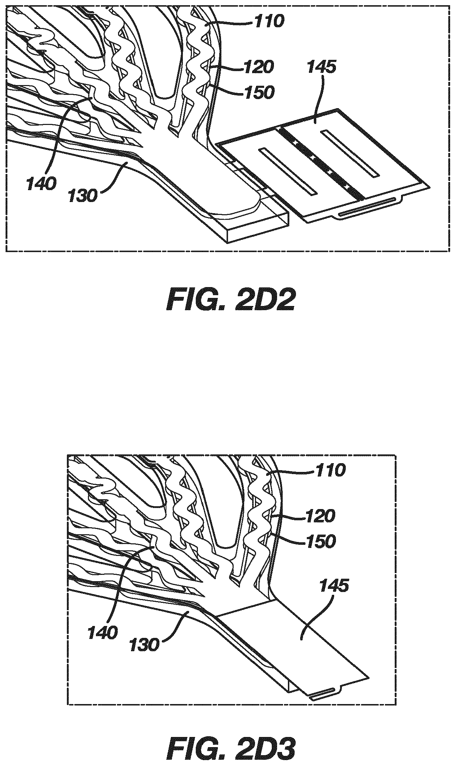

FIG. 2D2 is an illustration of a magnified view of the end effector of FIG. 2D1;
FIG. 2D3 is an illustration of a magnified view of the end effector of FIG. 2D1;

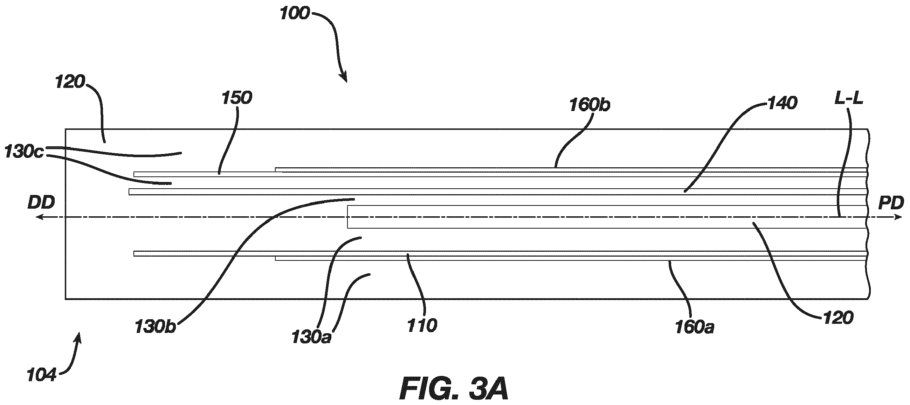

FIG. 3A is an illustration of a side view of an end effector encapsulated in a flexible material, in accordance with an embodiment of the present invention;

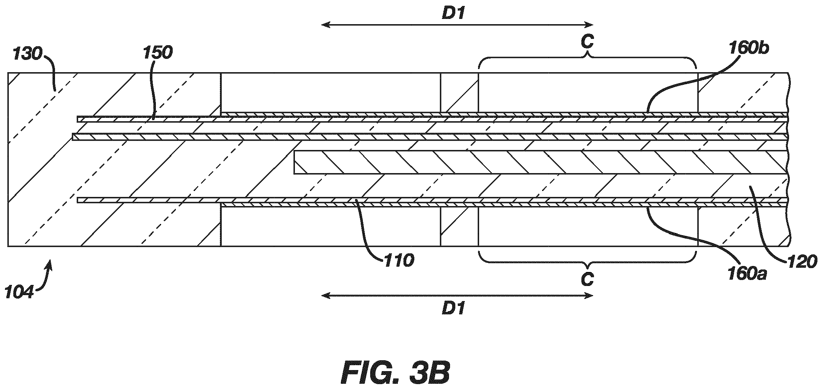

FIG. 3B illustrates a detailed view of the end effector of FIG. 3A;

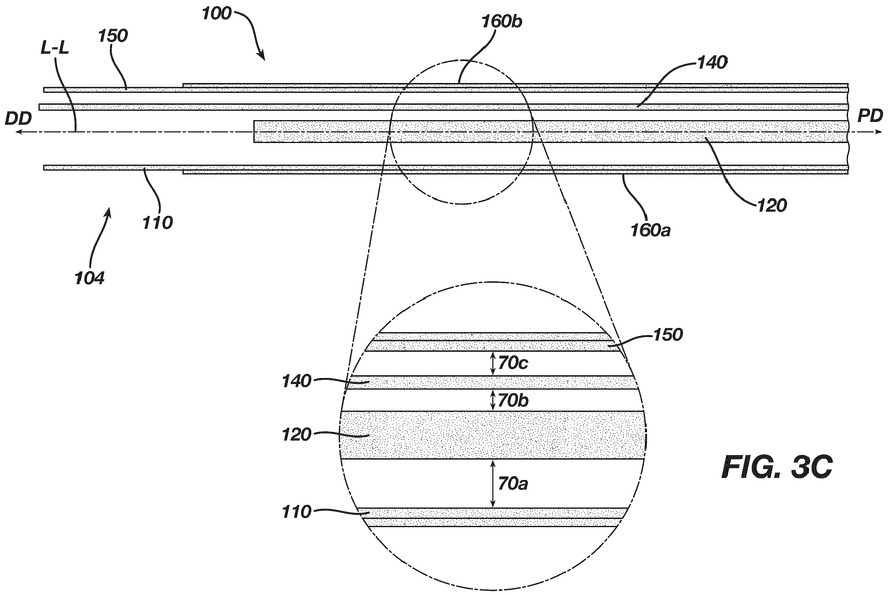

FIG. 3C is an illustration of the end effector of FIG. 3A without the flexible material;

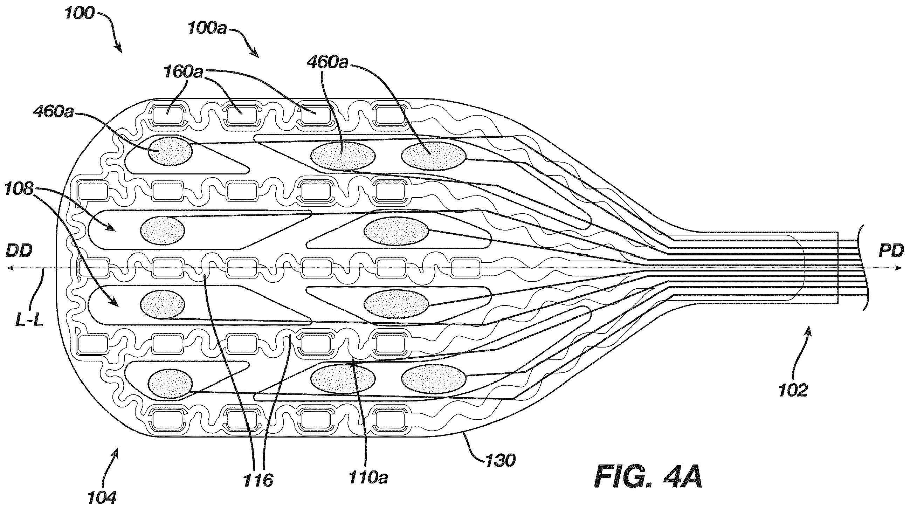

FIG. 4A is an illustration of a top view of the end effector, in accordance with an embodiment of the present invention;

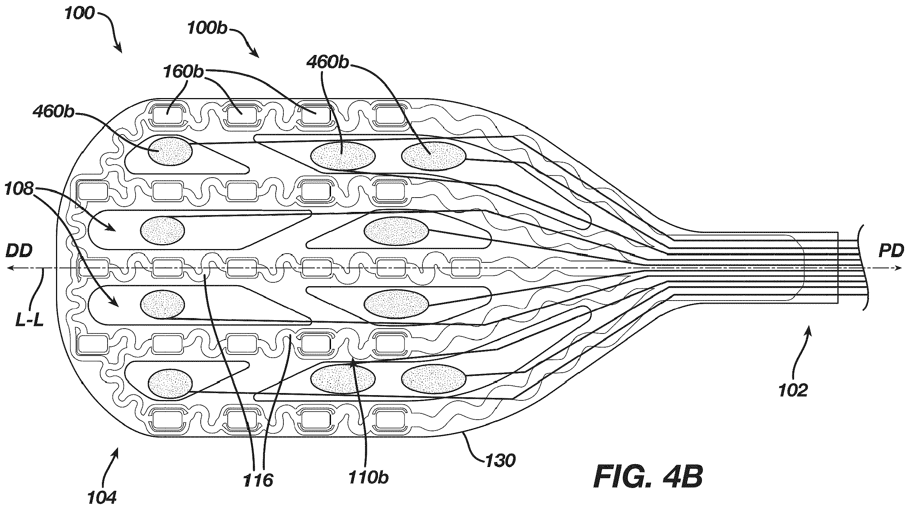

FIG. 4B is an illustration of a bottom view of the end effector, in accordance with an embodiment of the present invention;

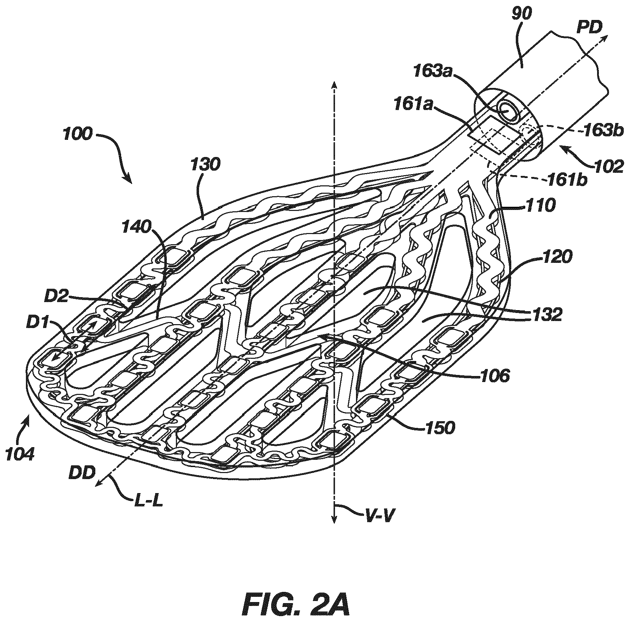

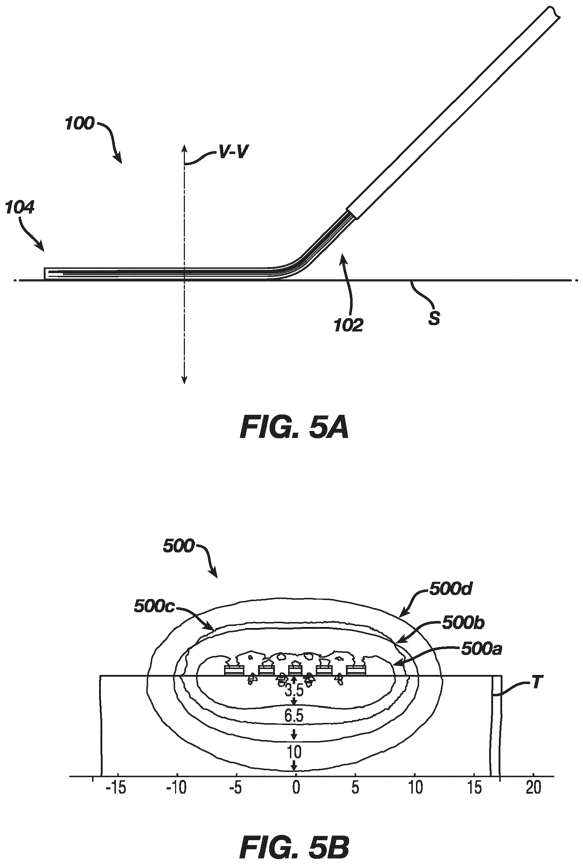

FIG. 5A is an illustration of the end effector of FIG. 2A in a typical use scenario;
FIG. 5B is an illustration of the voltage gradient during ablation with the electrodes of the end effector as ablation electrodes; and

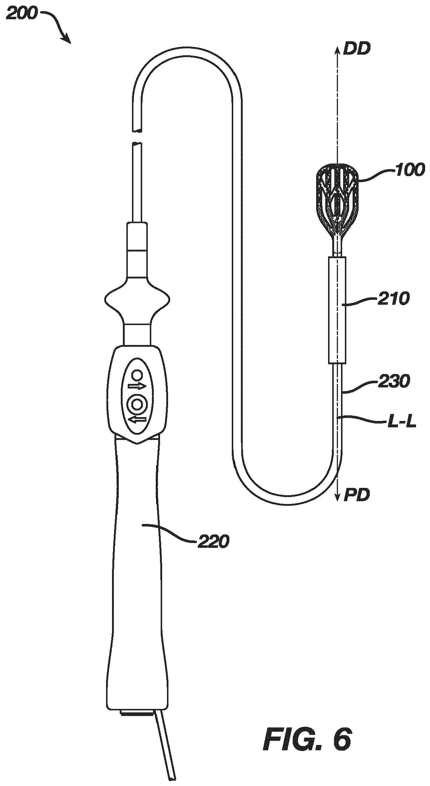

FIG. 6 is an illustration of a catheter assembly, in accordance with an embodiment of the present invention.

### DETAILED DESCRIPTION

To enhance the function of mapping and ablation catheters mentioned in the background section, the present disclosure relates to multilayered end effectors that enhance various aspects of mapping and ablation catheter performance, including, but not limited to: mapping resolution, electrode contact with the target anatomy, delivery of the end effector to the target anatomy, biocompatibility, end effector stiffness, and atraumaticity.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

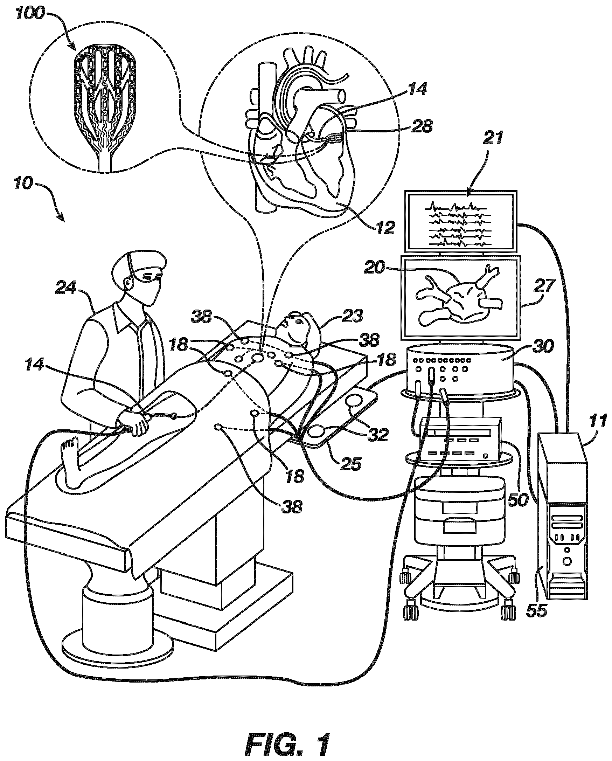

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a catheter shaft 90 with distal tip of catheter 14 (i.e., multilayered end effector 100) into contact with the heart wall for sensing a target site in heart 12. For ablation, physician 24 would similarly bring a distal end of an ablation catheter to a target site for ablating.

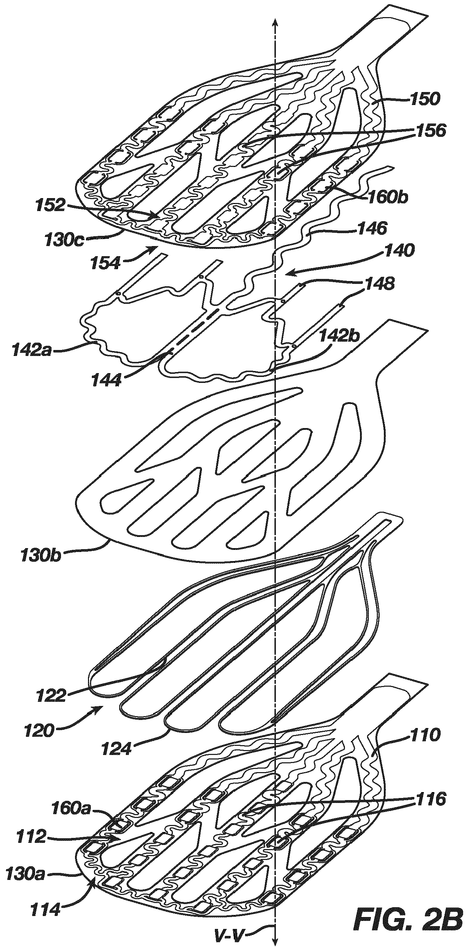

Catheter 14 is an exemplary catheter that includes one and preferably multiple electrodes 26 optionally distributed over end effector 100 coupled to a catheter shaft 90 and configured to sense the IEGM signals as described in more detail below. Catheter 14 may additionally include a position sensor (as shown in FIG. 2B) embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated by reference in their entirety into this application as if set forth in full and attached in the Appendix to priority application U.S. Provisional Patent Application No. 63/505,764 (Attorney Docket No. 253757.000380 BIO6846USPSP1) filed June 02, 2023.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182, each of which are incorporated by reference in their entirety into this application as if set forth in full and attached in the Appendix to priority application U.S. Provisional Patent Application No. 63/505,764 (Attorney Docket No. 253757.000380 BIO6846USPSP1) filed June 02, 2023.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

In order to achieve the desired stiffness, the mapping resolution, electrode contact with target anatomy, and conformity of the end effector disclosed herein to flat, curved, irregular and/or nonplanar tissue surfaces found in the target anatomy, the end effector has at least a framework, a flexible circuit layer, and first and second flexible non-conductive layers. As used herein, the term "flexible circuit" includes thin-film circuit, flexible printed circuit board, thin film deposition via lithography and etching processes on substrate such as polyimide or even nitinol substrate.

Reference is now made to FIG. 2A and FIG. 2B showing end effector 100 coupled to a catheter shaft 90. Importantly, the end effector 100 has an overall flat profile. FIG. 2B shows an exploded view of a multilayered end effector 100 of FIG. 2A for a catheter 200 (illustrated in FIG. 6). End effector 100 is illustrated being exploded in the orthogonal direction along vertical axis V-V. The end effector 100 can include a first flexible circuit 110, a framework 120, a first non-conductive flexible layer 130a, a second flexible circuit 150, and a second non-conductive flexible layer 130b. The first flexible circuit 110 can extend along a longitudinal axis L-L from a proximal portion 102 to a distal portion 104 of the end effector 100 and can include a first face 112 and a second face 114. Framework 120 can include a first side 122 and an opposite second side 124 and can extend along the longitudinal axis L-L generally parallel to the first flexible circuit 110 and with the first side 122 spaced apart from the first flexible circuit 110. First non-conductive flexible layer 130a can at least partially encapsulate the first flexible circuit 110 and the framework 120. Second flexible circuit 150 can be disposed on the second side 124 of the framework 120 and spaced apart from the framework 120, and the second flexible circuit 150 can have a first face 152 and a second face 154. Second non-conductive flexible layer 130b can at least partially encapsulate the second flexible circuit 150 and the framework 120.

In some examples, the first 110 and second 150 flexible circuit layers can be made primarily of polyimide. In other examples, it can be made of any of biocompatible polyimides, glass-reinforced epoxy laminate materials, copper, or graphene, alone or in combination. The first 110 and second 150 flexible circuit layers can include conductive traces.

The end effector 100 can further include a location sensing coil layer 140 including a plurality of coils 142 lying generally parallel to the framework 120 and separated from the framework 120 by a third non-conductive flexible layer 130c. Coils 142 can in some examples be coplanar. Relating to the location sensing coil layer 140, in some examples, as can be seen in FIG. 2B, the plurality of coils 142 can include two coils 142a, 142b joined together at a union 144 lying generally along the longitudinal axis L-L, and the location sensing coil layer 140 can further include a central spine 146 extending distally from the proximal portion 102 to the union 144. The central spine 146 can extend distally from the proximal portion 102 to the union 144 along a substantially sinusoidal path. The union 144 can be substantially straight and each of the two coils 142a, 142b otherwise extending along a substantially circular undulating path. The location sensing coil layer 140 can further include at least one elongate digit 148 extending from the plurality of coils 142. The at least one elongate digit 148 can extend generally parallel to the longitudinal axis L-L. The at least one elongate digit 148 can serve to modulate the stiffness of and to normalize the bending of end effector 100 by more evenly distributing the material of location sensing coil layer 140.

The end effector 100 can further include one or more first electrodes 160a affixed to the first face 112 of the first flexible circuit 110. Each of the one or more first electrodes 160a can have a contact surface C (shown in FIG. 3B) exposed through the first non-conductive flexible layer 130a to the ambient environment. It is noted that not all of the first electrodes 160a (or second electrodes 160b) are exposed through the non-conductive flexible layer 130a as these non-exposed electrodes can be used to sense far-field signals for noise reduction proximate the tissue contacting electrodes 160a or 160b. Similarly, far-field signals (including noise or artifacts) can be reduced or canceled out for the overall end effector with a reference electrode 161a disposed on one side near the proximal base 102 of the framework 100 such that the reference electrode 161a is not in contact with tissues and only with blood. Likewise, another reference electrode 161b can be disposed on the opposite facing surface of the framework 100 near the proximal base 102 so that the reference electrode is not in contact with tissue during mapping or recording (via tissue contact with electrodes 160). The reference electrodes 161a and 161b are preferably exposed to the ambient blood environment but can be encased in the polymer so that the reference electrodes are not exposed through the non-conductive layer. Irrigation can be provided with irrigation ports 163a (on one side) and 163b (on the other side) in fluid communication with an irrigation line (not shown) disposed in a catheter shaft 90. Instead of an irrigation line separate from the catheter shaft 90, a lumen can be formed via extrusion of the catheter shaft 90 to provide for a lumen channel. It is noted that port 163a or 163b can be configured to have a sufficient flow diverter characteristic for irrigation fluid to cover the mapping electrodes during irrigation flow so as to prevent or reduce thrombus formations.

The end effector 100 can further include one or more second electrodes 160b affixed to a first face 152 of the second flexible circuit 150. Each of the one or more second electrodes 160b can have a contact surface C (shown in FIG. 3B) exposed through the second non-conductive flexible layer 130b to the ambient environment. In some examples, the contact surface C of any of the first electrodes 160a and second electrodes 160b can be exposed by laser cutting through the respective first 130a and second 130b non-conductive polymer layer.

At least a portion of the first electrodes 160a can be axially aligned, orthogonal to the longitudinal axis L-L, with at least a position of the second electrodes 160b to define pairs of opposite facing electrodes. Electrodes 160 can sense tissue signals or transmit energy (AC or DC) from an energy generator to the tissues.

There can be provided a plurality of pairs of first electrodes 160a disposed on the first face 112 of the first flexible circuit 110, the electrodes of each pair of first electrodes 160a being spaced apart a first predetermined longitudinal distance Di and each pair of first electrodes 160a spaced apart from adjacent pairs of first electrodes 160a a second predetermined longitudinal distance D₂, the second predetermined longitudinal distance D₂ being greater than the first predetermined longitudinal distance Di.

The end effector 100 can further include a plurality of pairs of second electrodes 160b disposed on the first face 152 of the second flexible circuit 150, the electrodes of each pair of second electrodes 160b being spaced apart the first predetermined longitudinal distance Di and each pair of second electrodes 160b spaced apart from adjacent pairs of second electrodes 160b the second predetermined longitudinal distance D₂.

In some examples, there are about 92 electrodes 40 and about 46 pairs of first 160a and second 160b electrodes. In some examples, there are about 48 electrodes 40 and about 24 pairs of first 160a and second 160b electrodes. In some examples, there are about 64 electrodes 40 and about 32 pairs of first 160a and second 160b electrodes. In some examples, there are about 72 electrodes 40 and about 36 pairs of first 160a and second 160b electrodes. In some examples, there are about 98 electrodes 40 and about 49 pairs of first 160a and second 160b electrodes. Details of the spacing of the electrodes for each pair vs spacing between discrete sets of pairs of electrodes can be found in U.S. Provisional Patent Application S.N. 63/406,673 (Attorney Docket No. BIO6749USPSP3) filed on September 14, 2022 and incorporated by reference in its entirety into this application as if set forth in full and attached in the Appendix to priority application U.S. Provisional Patent Application No. 63/505,764 (Attorney Docket No. 253757.000380 BIO6846USPSP1) filed June 02, 2023.

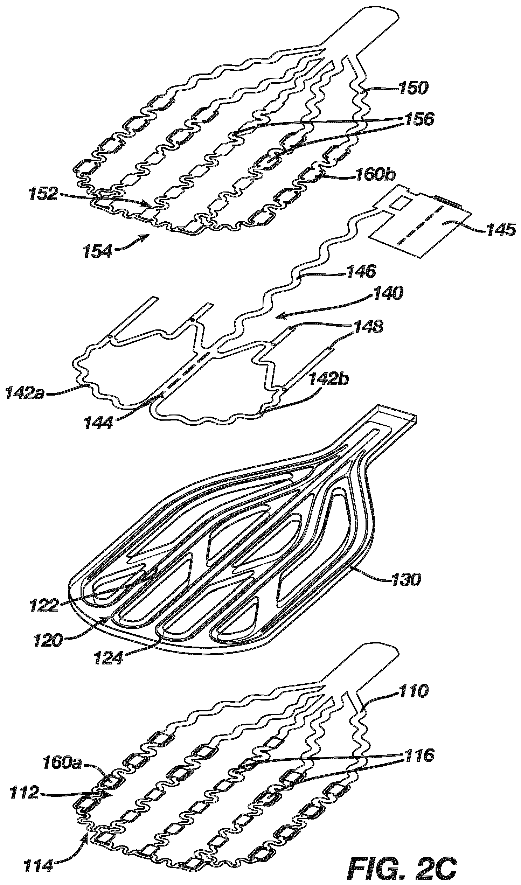

In FIG. 2C, it can be seen that a proximal location sensor 145 can be provided in which the location sensor can be folded flat or rolled to conform to the cylindrical form of the catheter shaft 90. Proximal location sensor 145 is shown as part of location sensing coil layer 140 in FIG. 2C.

FIG. 2D1, instead of proximal location sensor 145 being part of location sensing coil layer 140, a proximal location sensor 145 can be provided as part of first flexible circuit 110 and also as part of second flexible circuit 150. Additionally, two separate proximal location sensors 145 can be provided, one being part of the first flexible circuit 110 and the other being part of the second flexible circuit 150. Alternatively, three separate proximal location sensors in the form of the flexible circuit described and illustrated herein can be disposed 120 degrees about the longitudinal axis of the shaft 90 to provide a triple-axis-sensor (TAS). In some examples, location sensor 145 can be a single axis sensor (SAS).

In FIGs. 2D2 and 2D3, proximal location sensor 145 is shown unfolded (FIG. 2D2) and folded (FIG. 2D3) to conform flatly to end effector 100. In this example, when folded, location sensor 145 is not necessarily coplanar with any of first flexible circuit 110, framework 140, or second flexible circuit 150. Location sensor 145 can be embedded in flexible non-conductive material 130. Alternatively, location sensor 145 can be folded after other components, such as first flexible circuit 110 and second flexible circuit 150, are encapsulated by flexible non-conductive material 130 and thus would reside outside of flexible non-conductive material 140. Proximal location sensor 145 can also be rolled to form a cylinder, as discussed above in relation to FIG. 2C.

In some examples as shown in FIGs. 3A-3B, pairs of opposite facing electrodes can be aligned generally parallel to the longitudinal axis L-L. In some examples, pairs of opposite facing electrodes can be aligned generally transverse to the longitudinal axis L-L. Pairs of opposite facing electrodes can be utilized to cancel noise, with one electrode sensing tissue directly and the opposite electrode sensing far-field signals which typically may include noise, as is appreciated by those skilled in the pertinent art.

Continuing to reference FIGs. 3A-3C showing end effector 100, which in this example is provided for a mapping catheter 200. End effector 100 can be understood in other words as including a first flexible circuit 110 having a first face 112 and a second face 114 and extending along longitudinal axis L-L from a proximal portion 102 to a distal portion 104 of the end effector 100. End effector 100 can further include a framework 120 extending along the longitudinal axis L-L generally parallel to the first flexible circuit 110 and separated from the first flexible circuit 110 by a first orthogonal gap 70a orthogonal to the longitudinal axis L-L, and a second flexible circuit 150 having a first face 152 and a second face 154 and extending along the longitudinal axis L-L from the proximal portion 102 to the distal portion 104.

End effector 100 can further include a location sensing coil layer 140 having a plurality of coils 142 lying generally parallel to the framework 120 and separated from the framework 120 by a second orthogonal gap 70b. The second flexible circuit 150 can be separated from the location sensing coil layer 140 by a third orthogonal gap 70c.

Importantly, first 70a, second 70b, and third 70c orthogonal gaps function in part to allow the first flexible circuit 110, the framework 120, the location sensing coil layer 140, and the second flexible circuit 150 to flex and, to a limited extent, slip past one another so as to allow the end effector 100 to better conform to the target anatomy.

As shown in FIG. 3C, the first orthogonal gap 70a, the second orthogonal gap 70b, and the third orthogonal gap 70c can be filled with a flexible non-conductive material 130, and the first face 112 of the first flexible circuit 110 and the first face of the second flexible circuit 150 being coated with the flexible non-conductive material 130. In other words, the first flexible circuit 110, the framework 120, the location sensing coil layer 140, and the second flexible circuit 150 can each be suspended in the flexible non-conductive material 130 such that each of the first flexible circuit 110, the framework 120, the location sensing coil layer 140, and the second flexible circuit 150 are separated from one another.

This nonconductive flexible material 130 also serves to enhance the atraumaticity of the end effector 100 and to protect the subject from sharp edges.

End effector 100 can further include a first plurality of electrodes 160a being disposed in the flexible non-conductive material 130 and having a respective plurality of contact surfaces C being exposed to the ambient environment through the flexible non-conductive material 130. The first plurality of electrodes 160a can be exposed to the ambient environment through a plurality of holes in the flexible non-conductive material 130. In some examples, the first plurality of electrodes 160a disposed on the first flexible circuit 110.

In some examples, such as that shown in FIG. 4A, the first flexible circuit 110, the framework 120, and the second flexible circuit 150 can define a forked structure 100a having a plurality of tines 106, with some of the one or more first electrodes being first ablation electrodes 460a disposed between said tines 106. The flat profile of framework 120 can be formed from a planar or cylindrical stock of material using any suitable method. For example, the framework 120 can be formed by cutting, laser cutting, stamping, etc.

The one or more first electrodes can include first mapping electrodes 160a disposed on the first flexible circuit 110 and first ablation electrodes 460a disposed in the first non-conductive flexible layer 130a and having a contact surface C exposed through the first non-conductive flexible layer 130a to the ambient environment. In some examples, the first ablation electrodes 460a can be substantially coplanar with the first flexible circuit 110 and exposed to the ambient environment as previously described. Alternatively, all or some first ablation electrodes 460a can be disposed on first flexible circuit 110 and all or some of the first mapping electrodes 160a can be disposed in the first non-conductive flexible layer 130a.

FIG. 4B shows the side of end effector 100 opposite that shown in FIG. 4A, with some of the one or more second electrodes being second ablation electrodes 460b disposed between said tines 106. The one or more second electrodes can include second mapping electrodes 160b disposed on the second flexible circuit 150 and second ablation electrodes 460b disposed in the second non-conductive flexible layer 130b. In some examples, the second ablation electrodes 460b can be substantially coplanar with the second flexible circuit 150 and exposed to the ambient environment as previously described. Alternatively, all or some of the second ablation electrodes 460b can be disposed on second flexible circuit 150 and all or some of the second mapping electrodes 160b can be disposed in the second non-conductive flexible layer 130b.

In other examples, the first flexible circuit 110 can have a planar shape 110a having a plurality of tines 116 with the first plurality of electrodes 160a lying coplanar with the first flexible circuit 110 and being disposed between the tines 116 of the first flexible circuit 110.

In some examples, the second plurality of electrodes 160b can be disposed on the second flexible circuit 150. In other examples, the second flexible circuit 150 can have a planar shape 150a including a plurality of tines 156. The second plurality of electrodes 160b can be coplanar with the second flexible circuit 150 and disposed between the tines 156 of the second flexible circuit 150.

The first non-conductive flexible layer 130a, the second non-conductive flexible layer 130b, and the third non-conductive flexible layer 130c, as well as flexible non-conductive material 130 can also be thought of as a contiguous mass of flexible, non-conductive material 130. Stated in other words, effector 100 can include a flexible circuit extending generally parallel to a longitudinal axis L-L of the end effector 100, a plurality of electrodes electrically connected to the flexible circuit, and a framework 120 extending along the longitudinal axis L-L generally parallel to the flexible circuit, with the contiguous mass of flexible non-conductive material 130 separating the flexible circuit and the framework 120 from one another and at least partially electrically insulating the flexible circuit and the framework 120. The flexible circuit can include a first flexible circuit 110 and a second flexible circuit 150, each having a configuration as previously described.

In some examples, the flexible non-conductive material 130 can include biocompatible silicone. In other examples, the flexible non-conductive material 130 can include biocompatible flexible polymers, thermoelastic polymer materials, and dielectric materials. The flexible non-conductive material 130 can optionally be colored to facilitate laser cutting.

The contiguous mass of flexible, non-conductive material 130 can have portions 132 removed. Portions 132 can also be formed in one or all of the flexible circuit 110 and the framework 120. Portions 132 can pass through all layers of the multi-layered end effector 100, or just some of the layers. Portion 132 can be included to increase the ability of the end effector 100 to fold or bend into a reduced delivery configuration to allow the end effector 100 to be passed through a delivery catheter.

The portions 132 lying inside of the coils 142a, 142b can be removed in order to make the end effector 100 more permissive to flexion while maintaining the geometrical integrity and sensing ability of the coils 142a, 142b. Additionally, in some examples, the framework 120 can be shaped to make the end effector 100 more permissive to flexion while maintaining the geometrical integrity and sensing ability of the location sensing coil layer 140.

The location sensing coil layer 140 can be used in conjunction with one or more magnetic field generators (not shown) to determine the position of the end effector 100 within the subject via triangulation. Electromagnetic location sensing technique is described in US Patent Nos. 5,391,199; 5,443,489; 5,558,091; 6,172,499; 6,690,963; and 6,788,967 which are incorporated by reference in their entirety into this application as if set forth in full and attached in the Appendix to priority application U.S. Provisional Patent Application No. 63/505,764 (Attorney Docket No. 253757.000380 BIO6846USPSP1) filed June 02, 2023.

In some examples, the end effector 100 can further include a location sensing coil layer 140 separated from the flexible circuit and the framework 120 by a portion of the contiguous mass of flexible non-conductive material 130. Alternatively, in some examples, the framework 120 can be used as the location sensing coil layer 140.

In some examples, the electrodes 160 can be disposed in the contiguous mass of flexible non-conductive material 130 and exposed to the ambient environment through a plurality of respective holes 132 in the contiguous mass of flexible non-conductive material 130.

In some examples, the second flexible circuit 150 can be disposed on a side of the framework 120 opposite the first flexible circuit 110, and the plurality of electrodes can include a first plurality of electrodes 160a electrically connected to the first flexible circuit 110 and a second plurality of electrodes 160b electrically connected to the second flexible circuit 150. The contiguous mass of flexible non-conductive material 130 can separate the second flexible circuit 150 and the framework 120 from one another and at least partially insulating the second flexible circuit 150 and the framework 120.

In some examples, the first plurality of electrodes 160a and the second plurality of electrodes 160b can be exposed to the ambient environment through the plurality of respective holes 132 in the contiguous mass of flexible non-conductive material 130.

In some examples, such as that shown in FIG. 5A, the end effector 100 can remain in contact against a generally planar surface S with a force applied to the proximal portion 102 of the end effector 100 in a vertical axis V-V with respect to the generally planar surface S. The end effector 100 can remain in contact against generally planar surface S with a force applied to the proximal portion 102 of the end effector 100 in a vertical axis V-V with respect to the generally planar surface S and a force applied generally parallel to the generally planar surface S. Physician 24 can also apply a torque to the end effector 100 via a tubular member 230 (shown in FIG. 6 and described in more detail below), thus prompting end effector 100 to lay flat against planar surface S. End effector 100 can also be pushed flat against generally planar surface S with a force applied generally parallel to the longitudinal axis L-L. End effector 100 can be made to lay flat against generally planar surface S via the actuation of pull wires by physician 24 as physician 24 drags end effector 100 across the generally planar surface S. Details of the manipulation of end effectors via pull wires are described in U.S. Patent Publication No. 2021/0369339, which is incorporated by reference in its entirety into this application as if set forth in full and attached in the Appendix to priority application U.S. Provisional Patent Application No. 63/505,764 (Attorney Docket No. 253757.000380 BIO6846USPSP1) filed June 02, 2023. FIG. 5B demonstrates the ability of the end effector 100 to generate bipolar ablation between electrodes on opposite sides of the end effector 100 as well as between electrodes on the same side of the end effector 100. FIG. 5B shows an illustration of a simulation 500 of bipolar ablation generated between the tissue contacting ablation electrodes, which in this case are ablation electrodes 460a and the non-tissue contacting ablation electrodes, which in this case are ablation electrodes 460b on the opposite side of the end effector. In FIG. 5B, rings 500a, 500b, 500c, and 500d represent voltages of 800v, 1200v, 1800v, and 1800v respectively, and the penetration depth into tissue T, shown in millimeters.

In some examples, the first flexible circuit 110, the second flexible circuit 150, and the framework 120 can each be configured to slip within the non-conductive material 130 with respect to one another upon a bending of the end effector 100 as illustrated in FIG. 5A.

The present disclosure provides a catheter assembly 200 as shown in FIG. 6 which can include a tubular member 230 extending along a longitudinal axis L-L and configured to deliver end effector 100 to an out of a sheath 210. A physician 24 can manipulate the catheter 200 with handle 220. Appropriate examples for catheter assembly 200 and its subcomponents such as handle 220, sheath 210, tubular member 230, and others not mentioned herein are described in US Patent publication No. 2021/0369339, which is incorporated by reference in its entirety into this application as if set forth in full and attached in the Appendix to priority application U.S. Provisional Patent Application No. 63/505,764 (Attorney Docket No. 253757.000380 BIO6846USPSP1) filed June 02, 2023.

The disclosed technology described herein can be further understood according to the following clauses:
Clause 1: A multilayered end effector for a catheter, the end effector comprising: a first flexible circuit extending along a longitudinal axis from a proximal portion to a distal portion of the end effector, the first flexible circuit comprising a first face and a second face; a framework comprising a first side and an opposite second side, and extending along the longitudinal axis generally parallel to the first flexible circuit and with the first side spaced apart from the first flexible circuit such that there is no physical contact between the framework and the flexible circuit; a first non-conductive flexible layer at least partially encapsulating the first flexible circuit and the framework; a second flexible circuit disposed on the second side of the framework and spaced apart from the framework, the second flexible circuit having a first face and a second face; and a second non-conductive flexible layer at least partially encapsulating the second flexible circuit and the framework.
Clause 2: The end effector of clause 1, further comprising: a location sensing coil layer comprising a plurality of coils suitably oriented and preferably disposed generally parallel to the framework and separated from the framework by a third non-conductive flexible layer.
Clause 3: The end effector according to any of clauses 1-2, further comprising: one or more first electrodes affixed to the first face of the first flexible circuit, each of the one or more first electrodes having a contact surface exposed through the first non-conductive flexible layer to the ambient environment and some of the one or more electrodes are not exposed through the non-conductive flexible layer
Clause 4: The end effector of any of clauses 1-3, further comprising one or more second electrodes affixed to a first face of the second flexible circuit, each of the one or more second electrodes having a contact surface exposed through the second non-conductive flexible layer to the ambient environment.
Clause 5: The end effector of any of clauses 3-4, in which at least a portion of the first electrodes are axially aligned, orthogonal to the longitudinal axis, with at least a position of the second electrodes to define pairs of opposite facing electrodes.
Clause 6: The end effector of clause 5, in which pairs of opposite facing electrodes are aligned generally parallel to the longitudinal axis.
Clause 7: The end effector of clause 5 or clause 6, in which pairs of opposite facing electrodes are aligned generally transverse to the longitudinal axis.
Clause 8: The end effector of any of clauses 1-2, further comprising a plurality of pairs of first electrodes disposed on the first face of the first flexible circuit, the electrodes of each pair of first electrodes being spaced apart a first predetermined longitudinal distance and each pair of first electrodes spaced apart from adjacent pairs of first electrodes a second predetermined longitudinal distance, the second predetermined longitudinal distance being greater than the first predetermined longitudinal distance.
Clause 9: The end effector of clause 8, further comprising a plurality of pairs of second electrodes disposed on the first face of the second flexible circuit, the electrodes of each pair of second electrodes being spaced apart the first predetermined longitudinal distance and each pair of second electrodes spaced apart from adjacent pairs of second electrodes the second predetermined longitudinal distance.
Clause 10: The end effector of clause 3, further comprising one or more first electrodes disposed in the first non-conductive flexible layer and having a contact surface exposed through the first non-conductive flexible layer to the ambient environment.
Clause 11: The end effector of clause 10, the one or more first electrodes being substantially coplanar with the first flexible circuit.
Clause 12: The end effector of clause 11, the first flexible circuit, the framework, and the second flexible circuit defining a forked structure having a plurality of tines, the one or more first electrodes being disposed between said tines.
Clause 13: The end effector of clause 3, further comprising one or more second electrodes disposed in the second non-conductive flexible layer, each of the one or more second electrodes having a contact surface exposed through the second non-conductive flexible layer to the ambient environment.
Clause 14: The end effector of clause 13, the one or more second electrodes being substantially coplanar with the second flexible circuit.
Clause 15: The end effector of clause 14, the first flexible circuit, the framework, and the second flexible circuit defining a forked structure having a plurality of slots, the one of more second electrodes being disposed in said slots.
Clause 16: The end effector of any of clauses 2-15, the plurality of coils comprising two coils joined together at a union lying generally along the longitudinal axis, and the location sensing coil layer further comprising: a central spine extending distally from the proximal portion to the union.
Clause 17: The end effector of clause 16, the central spine extending distally from the proximal portion to the union along a substantially sinusoidal path.
Clause 18: The end effector of an of clauses 16-17, the union being straight and each of the two coils otherwise extending along a substantially circular undulating path.
Clause 19: The end effector of any of clauses 16-18, the location sensing coil layer further comprising at least one elongate digit extending from the plurality of coils.
Clause 20: The end effector of any of clauses 1-19, in which the end effector remains in contact against a generally planar surface with a force applied to the proximal portion of the end effector in a vertical axis with respect to the generally planar surface.
Clause 21: The end effector of any of clauses 1-19, in which the end effector remains in contact against a generally planar surface with a force applied to the proximal portion of the end effector in a vertical axis with respect to the generally planar surface and a force applied generally parallel to the generally planar surface.
Clause 22: The end effector of any of clauses 3-15, the one or more first electrodes comprising one or more mapping electrodes.
Clause 23: The end effector of any of clauses 3-15, the one or more first electrodes comprising one or more ablation electrodes.
Clause 24: The end effector of any of clauses 4-15, the one or more second electrodes comprising one or more mapping electrodes.
Clause 25: The end effector of any of clauses 4-15, the one or more second electrodes comprising one or more ablation electrodes.
Clause 26: An end effector for a mapping catheter, the end effector comprising: a first flexible circuit comprising a first face and a second face and extending along a longitudinal axis from a proximal portion to a distal portion of the end effector; a framework extending along the longitudinal axis generally parallel to the first flexible circuit and separated from the first flexible circuit by a first orthogonal gap orthogonal to the longitudinal axis; and a second flexible circuit comprising a first face and a second face and extending along the longitudinal axis from the proximal portion to the distal portion.
Clause 27: The end effector of clause 26, the end effector further comprising: a location sensing coil layer comprising a plurality of coils suitably oriented and preferably disposed generally parallel to the framework and separated from the framework by a second orthogonal gap, the second flexible circuit separated from the location sensing coil layer by a third orthogonal gap.
Clause 28: The end effector of clause 27, the first orthogonal gap, the second orthogonal gap, and the third orthogonal gap being filled with a flexible non-conductive material and the first face of the first flexible circuit and the first face of the second flexible circuit being coated with the flexible non-conductive material.
Clause 29: The end effector of clause 28, further comprising a first plurality of electrodes being disposed in the flexible non-conductive material and having a respective plurality of contact surfaces being exposed to the ambient environment through the flexible non-conductive material.
Clause 30: The end effector of clause 29, the first plurality of electrodes disposed on the first flexible circuit.
Clause 31: The end effector of clause 29, the first flexible circuit having a planar shape comprising a plurality of tines, the first plurality of electrodes coplanar with the first flexible circuit and disposed between the tines of the first flexible circuit.
Clause 32: The end effector of clause 31, further comprising a second plurality of electrodes being disposed in the flexible non-conductive material and having a respective plurality of contact surfaces being exposed to the ambient environment through the flexible non-conductive material.
Clause 33: The end effector of clause 32, the second plurality of electrodes disposed on the second flexible circuit.
Clause 34: The end effector of clause 33, the second flexible circuit having a planar shape comprising a plurality of tines, the second plurality of electrodes coplanar with the second flexible circuit and disposed between the tines of the second flexible circuit.
Clause 3 5: An end effector for a catheter, the end effector comprising: a flexible circuit extending generally parallel to a longitudinal axis of the end effector and comprising a first flexible circuit and a second flexible circuit; a plurality of electrodes electrically connected to the flexible circuit; a framework extending along the longitudinal axis generally parallel to the flexible circuit; and a contiguous mass of flexible non-conductive material separating the flexible circuit and the framework from one another and at least partially insulating the flexible circuit and the framework.
Clause 36: The end effector of clause 35 further comprising a location sensing coil layer separated from the flexible circuit and the framework by the contiguous mass of flexible non-conductive material.
Clause 37: The end effector of clause 36, the electrodes being disposed in the contiguous mass of flexible non-conductive material and exposed to the ambient environment through a plurality of respective holes in the contiguous mass of flexible non-conductive material.
Clause 38: The end effector according to any of clauses 35-37, the second flexible circuit disposed on a side of the framework opposite the first flexible circuit, and the plurality of electrodes comprising a first plurality of electrodes electrically connected to the first flexible circuit and a second plurality of electrodes electrically connected to the second flexible circuit, the contiguous mass of flexible non-conductive material separating the second flexible circuit and the framework from one another and at least partially insulating the second flexible circuit and the framework.
Clause 39: The end effector of any of clauses 37-38, the first plurality of electrodes and the second plurality of electrodes being exposed to the ambient environment through the plurality of respective holes in the contiguous mass of flexible non-conductive material.
Clause 40: The end effector of any of clause 39, the first flexible circuit, the second flexible circuit, and the framework each being configured to slip within the non-conductive material with respect to one another upon a bending of the end effector.

## Claims

1. A multilayered end effector for a catheter, the end effector comprising:
a first flexible circuit extending along a longitudinal axis from a proximal portion to a distal portion of the end effector, the first flexible circuit comprising a first face and a second face;
a framework comprising a first side and an opposite second side, and extending along the longitudinal axis generally parallel to the first flexible circuit and with the first side spaced apart from the first flexible circuit;
a first non-conductive flexible layer at least partially encapsulating the first flexible circuit and the framework;
a second flexible circuit disposed on the second side of the framework and spaced apart from the framework, the second flexible circuit having a first face and a second face; and
a second non-conductive flexible layer at least partially encapsulating the second flexible circuit and the framework.

2. The end effector of claim 1, further comprising:
a location sensing coil layer comprising a plurality of coils disposed generally parallel to the framework and separated from the framework by a third non-conductive flexible layer.

3. The end effector according to claim 2, further comprising:
one or more first electrodes affixed to the first face of the first flexible circuit, each of the one or more first electrodes having a contact surface exposed through the first non-conductive flexible layer to the ambient environment.

4. The end effector according to claim 3, further comprising one or more second electrodes affixed to a first face of the second flexible circuit, each of the one or more second electrodes having a contact surface exposed through the second non-conductive flexible layer to the ambient environment.

5. The end effector according to claim 4, in which at least a portion of the first electrodes are axially aligned, orthogonal to the longitudinal axis, with at least a position of the second electrodes to define pairs of opposite facing electrodes.

6. The end effector according to claim 2, the plurality of coils comprising two coils joined together at a union lying generally along the longitudinal axis, and the location sensing coil layer further comprising:
a central spine extending distally from the proximal portion to the union, optionally wherein the location sensing coil layer further comprising at least one elongate digit extending from the plurality of coils.

7. The end effector according to claim 1, further comprising a plurality of pairs of first electrodes disposed on the first face of the first flexible circuit, the electrodes of each pair of first electrodes being spaced apart a first predetermined longitudinal distance and each pair of first electrodes spaced apart from adjacent pairs of first electrodes a second predetermined longitudinal distance, the second predetermined longitudinal distance being greater than the first predetermined longitudinal distance.

8. The end effector of claim 1, further comprising one or more first electrodes disposed in the first non-conductive flexible layer and having a contact surface exposed through the first non-conductive flexible layer to the ambient environment, optionally further comprising one or more second electrodes disposed in the second non-conductive flexible layer, each of the one or more second electrodes having a contact surface exposed through the second non-conductive flexible layer to the ambient environment.

9. The end effector according to claim 1, in which the end effector remains in contact against a generally planar surface with a force applied to the proximal portion of the end effector in a vertical axis with respect to the generally planar surface.

10. An end effector for a mapping catheter, the end effector comprising:
a first flexible circuit comprising a first face and a second face and extending along a longitudinal axis from a proximal portion to a distal portion of the end effector;
a framework extending along the longitudinal axis generally parallel to the first flexible circuit and separated from the first flexible circuit by a first orthogonal gap orthogonal to the longitudinal axis; and
a second flexible circuit comprising a first face and a second face and extending along the longitudinal axis from the proximal portion to the distal portion.

11. The end effector of claim 10, the end effector further comprising:
a location sensing coil layer comprising a plurality of coils disposed generally parallel to the framework and separated from the framework by a second orthogonal gap, the second flexible circuit separated from the location sensing coil layer by a third orthogonal gap,
optionally the first orthogonal gap, the second orthogonal gap, and the third orthogonal gap being filled with a flexible non-conductive material and the first face of the first flexible circuit and the first face of the second flexible circuit being coated with the flexible non-conductive material,
further optionally further comprising a first plurality of electrodes being disposed in the flexible non-conductive material and having a respective plurality of contact surfaces being exposed to the ambient environment through the flexible non-conductive material.

12. An end effector for a catheter, the end effector comprising:
a flexible circuit extending generally parallel to a longitudinal axis of the end effector and comprising a first flexible circuit and a second flexible circuit;
a plurality of electrodes electrically connected to the flexible circuit;
a framework extending along the longitudinal axis generally parallel to the flexible circuit; and
a contiguous mass of flexible non-conductive material separating the flexible circuit and the framework from one another and at least partially insulating the flexible circuit and the framework.

13. The end effector of claim 12 further comprising a location sensing coil layer separated from the flexible circuit and the framework by the contiguous mass of flexible non-conductive material.

14. The end effector of claim 13, the electrodes being disposed in the contiguous mass of flexible non-conductive material and exposed to the ambient environment through a plurality of respective holes in the contiguous mass of flexible non-conductive material.

15. The end effector according to claim 14, the second flexible circuit disposed on a side of the framework opposite the first flexible circuit, and the plurality of electrodes comprising a first plurality of electrodes electrically connected to the first flexible circuit and a second plurality of electrodes electrically connected to the second flexible circuit,
the contiguous mass of flexible non-conductive material separating the second flexible circuit and the framework from one another and at least partially insulating the second flexible circuit and the framework,
optionally the first plurality of electrodes and the second plurality of electrodes being exposed to the ambient environment through the plurality of respective holes in the contiguous mass of flexible non-conductive material.
